# DEMANDE DE BREVET EUROPEEN

(11) **EP 2 058 241 A1**
(43) Date de publication de la demande: **13.05.2009**
(21) Numéro de dépôt: 07405323.2
(22) Date de dépôt: 09.11.2007
(51) Int. Cl.: B65D 47/34, A61M 5/142, B05B 11/00

(54) **Enceinte étanche et procédé de fabrication de cette enceinte**

(71) Demandeur: Rochat, Jean-Denis, 1272 Genolier (CH)
(72) Inventeur: Rochat, Jean-Denis, 1272 Genolier (CH)
(74) Mandataire: Savoye, Jean-Paul

(57) **Abrégé**

Enceinte étanche dont la paroi comporte deux parties (1, 2; 11, 12), qui présentent des surfaces d'assemblage respectives (1a, 2a) et une partie intermédiaire (3, 13) présentant des surfaces d'assemblage (3a) complémentaires de celles des deux parties de paroi (1, 2; 11, 12) pour réunir de manière étanche les surfaces d'assemblage complémentaires les unes aux autres, les trois parties étant en polymère injecté. Ces trois parties (1, 2, 3; 11, 12, 13) sont en un polymère rigide, l'une d'entre elles au moins présentant au moins une surface de liaison permanente avec au moins un élément (4-7; 14-17) qui est en un élastomère thermoplastique ou en un élastomère silicone.

## Description

La présente invention se rapporte à une enceinte étanche dont la paroi comporte deux parties, qui présentent des surfaces d'assemblage respectives et une partie intermédiaire présentant des surfaces d'assemblage complémentaires de celles des deux parties de paroi pour réunir de manière étanche les surfaces d'assemblage complémentaires les unes aux autres, les trois parties étant en polymère injecté. L'invention se rapporte également à un procédé de fabrication de cette enceinte.

Il existe de nombreuses applications de ce type d'enceinte étanche renfermant des valves et un élément mobile alternativement apte à déplacer un liquide entre l'ouverture d'admission et l'ouverture de sortie. Une application très répandue est celle de la distribution par pulvérisation à travers une buse, d'un liquide contenu dans un récipient. Il peut s'agir de toute sorte de liquide distribué à travers un pulvérisateur, dans tous les domaines y compris dans le domaine médical pour les produits d'hygiène ou de traitement nasal notamment.

Il peut aussi s'agir d'une enceinte destinée à contrôler un écoulement de liquide par gravité et contenant une valve destinée à se fermer dès que la hauteur de la colonne de liquide descend au-dessous d'un seuil déterminé. Cette application est utilisée en particulier dans les conduits de perfusion pour empêcher l'entrée d'air à la fin de la perfusion.

L'enceinte étanche est généralement destinée à un usage limité dans le temps et jetable en sorte qu'il est important de réduire son coût de fabrication au maximum. L'existence d'éléments mobiles pour entraîner et/ou pour contrôler l'écoulement de fluide ne permet en principe pas de former l'enceinte étanche contenant cet élément mobile par injection de l'enceinte et du ou des élément mobiles en une seule matière injectée, les différentes parties de l'enceinte et l'élément mobile devant présenter des propriétés incompatibles les unes avec les autres.

En général, au moins une partie de la paroi de l'enceinte devra être relativement rigide, alors que les éléments pour interrompre temporairement l'écoulement de liquide et l'élément pour entraîner le liquide de l'ouverture d'admission à l'ouverture de sortie devront être souples.

Il en est de même pour les joints d'étanchéité entre les parties de la paroi de l'enceinte si il y en a.

Dans le cas d'un boîtier séparé de manière étanche en deux cavités, les joints d'étanchéité devront être compressibles, donc en un autre matériau que celui des parois de l'enceinte.

Actuellement, il n'existe pas de problème technique pour réaliser les enceintes étanches susmentionnées. Le problème est celui du coût, dans la mesure où il est nécessaire de fabriquer plusieurs pièces et de les assembler. Même avec des robots, dès que l'on doit fabriquer plusieurs pièces séparément et les assembler, on se trouve face à un processus de fabrication complexe et donc plus coûteux.

Le but de la présente invention est de remédier, au moins en partie, aux inconvénients susmentionnés.

A cet effet, cette invention a tout d'abord pour objet une enceinte étanche selon la revendication 1.

Elle a également pour objet un procédé de fabrication de cette enceinte étanche selon la revendication 8.

L'enceinte étanche selon l'invention avec son élément pour interrompre temporairement ou non la communication entre les ouvertures d'admission et de sortie forme avantageusement une seule pièce, supprimant de ce fait les opérations habituelles d'assemblage, permettant de diminuer le coût de l'enceinte de manière très significative.

Le dessin annexé illustre, schématiquement et à titre d'exemple plusieurs formes d'exécution de l'enceinte étanche objet de la présente invention.
La figure 1 est une vue en élévation coupée d'une première forme d'exécution relative à une pompe;
la figure 2 est une vue en élévation coupée d'une deuxième forme d'exécution relative à une pompe manuelle;
la figure 3 est une vue en élévation coupée d'une troisième forme d'exécution relative à une pompe manuelle nasale;
la figure 4 est une vue en élévation coupée d'une quatrième forme d'exécution relative à un raccord Luer à valve;
la figure 5 est une vue en élévation coupée d'une cinquième forme d'exécution relative à un élément de filtrage;
la figure 6 est une vue en perspective dépliée de la forme d'exécution de la figure 1, après l'opération d'injection et avant celle d'assemblage.

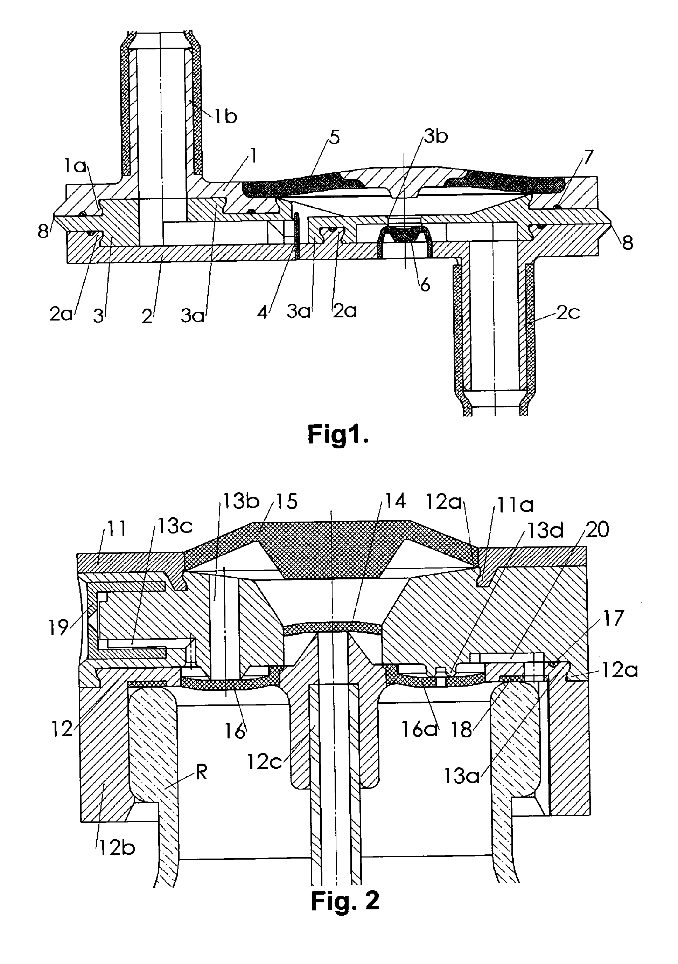

La figure 1 se rapporte à une enceinte étanche présentant la forme d'une pompe de perfusion ou de nutrition entérale comprenant trois parties, deux parties 1, 2 formant la paroi de l'enceinte et une partie intermédiaire 3 à laquelle les deux autres parties sont assemblées de façon étanche. A cet effet, la partie de paroi 1 comporte des surfaces d'assemblage 1a par enclenchement avec des surfaces d'assemblage 3a complémentaires de la partie médiane 3. De son côté la partie de paroi 2 comporte des surfaces d'assemblage par enclenchement 2a, complémentaires des surfaces d'assemblage 3a de la partie médiane 3.

La partie de paroi 1 présente un conduit d'admission 1b qui communique avec un premier compartiment de la pompe fermé par un clapet de valve 4 solidaire de l'autre partie de paroi 2. La partie de paroi 1 est solidaire d'une membrane de pompage 5, déformable de manière élastique, adjacente à un second compartiment de la pompe, ménagé entre la partie intermédiaire 3 et cette membrane de pompage 5. La partie intermédiaire comporte une ouverture 3b située en face du centre de la membrane de pompage 5. Cette ouverture est fermée, du côté opposé à la membrane de pompage 5, par un clapet de valve 6 solidaire de la partie de paroi 2. Cette partie de paroi 2 présente un conduit de sortie 2c qui communique avec l'ouverture 3b contrôlée par le clapet de valve 6. Des joints d'étanchéité 7 sont disposés entre les surfaces de la partie intermédiaire 3 adjacentes respectivement aux surfaces des parties de parois 1, 2, pressées l'une contre l'autre par les surfaces d'assemblage par enclenchement 1a, 2a, 3a.

En variante, il est aussi possible de réaliser une étanchéité satisfaisante à l'aides des surfaces d'assemblage par enclenchement 1a, 2a, 3a. Selon une autre variante, l'étanchéité peut aussi être obtenue par soudage par ultrasons.

Comme on peut le constater, une telle pompe doit comporter des parties relativement rigides pour former les parois de l'enceinte en particulier, et des parties souples pour former les clapets de valves 4, 6, la membrane de pompage 5 et les joints d'étanchéité 7. Pour réaliser ces parties de pompe, on utilise la technique de bi-injection qui consiste à injecter une première matière dans un moule, à enlever la partie supérieure du moule pour la remplacer par une autre partie supérieure conformée pour le moulage de la seconde matière.

Le recours à cette technique nécessite d'utiliser deux matériaux susceptibles d'être injectés. Il faut également que les surfaces du premier matériau, adjacentes au second matériau permettent une excellente adhérence. Pour le premier matériau injectable destiné à former les parties rigides de l'enceinte de pompage, on peut choisir par exemple un polypropylène ou un copolymère styrène-butadiène (SBC) tel que la K-resin^{®}. Pour le second matériau injectable on peut utiliser un élastomère thermoplastique (TPE), par exemple un polymère styrénique, une polyoléfine tel que le Santoprène^{®} ou un polyester tel que l'Hytrel^{®} ou l'Arnitel^{®}.

Pour le second matériau injectable, on peut aussi utiliser un matériau de la famille des caoutchoucs silicone liquides (LSR) aussi appelés élastomères silicone qui ont l'avantage d'être plus stables dans le temps que les TPE. L'élastomère silicone peut être utilisé sous la forme d'un matériau homogène ou sous forme de mousse.

Le moule d'injection de l'enceinte de pompage illustrée par la figure 1 ne comporte pas de tiroir. Comme on peut le constater sur la figure 1, la membrane de pompage 5 comporte une partie annulaire en TPE ou en LSR alors que la partie centrale est en polypropylène ou en SBC.

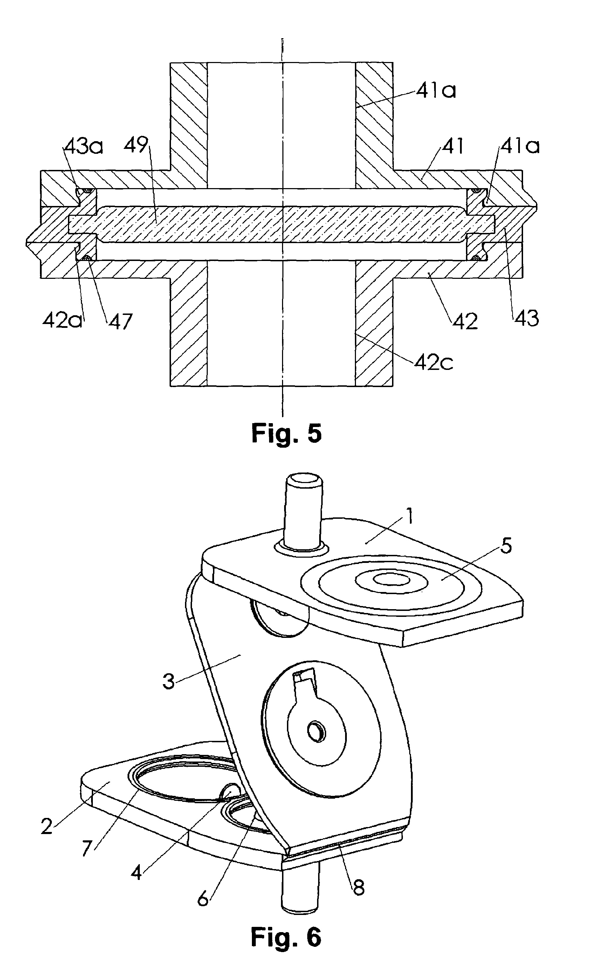

Avantageusement, comme illustré par la figure 6, les trois parties 1-3 de l'enceinte de pompage sont injectées ensemble, reliées les unes aux autres par des éléments de liaison pliables 8, permettant de les superposer par un pliage en Z avant de les assembler par une simple pression apte à provoquer l'enclenchement des surfaces d'assemblage 1a, 2a, 3a les unes dans les autres. Cette opération de pliage peut être réalisée sans difficulté par un robot.

Ce pliage en Z de trois parties reliées les unes aux autres par les éléments de liaison pliables 8 nécessite un mouvement de translation qui est plus avantageux à réaliser par un robot qu'un pliage par rotation qui serait nécessaire si l'enceinte était réalisée en deux parties au lieu de trois.

La forme d'exécution de la figure 2 est une adaptation de la pompe de la figure 1 à un distributeur de liquide contenu dans un récipient R dont seul le goulot est illustré sur la figure 2. Dans cette forme d'exécution, la partie 11 de la paroi de l'enceinte solidaire de la membrane de pompage 15 déformable de manière élastique, ne comporte pas de conduit d'admission ni aucun autre conduit. Le conduit d'admission 12c est venu de moulage par injection avec l'autre partie 12 de la paroi de l'enceinte, qui comporte aussi une partie tubulaire 12b pour la fixation de l'enceinte de pompage sur goulot du récipient R.

La partie de paroi 12 comporte deux valves 16 et 16a et un joint annulaire 18 pour faire l'étanchéité avec le récipient R. La partie médiane 13 comporte un clapet de valve 14 pour fermer le conduit d'admission 12c, un conduit 13b qui traverse la partie médiane 13 dans le sens de son épaisseur et dont une extrémité est fermée par le clapet 16. Un second conduit coudé 13c sort latéralement de l'enceinte, entre la partie intermédiaire 13 et la partie de paroi 12. Une buse rapportée 19 est fixée latéralement dans un espace ménagé en partie dans la partie médiane 13 et dans la partie de paroi 12, cet espace englobant l'extrémité de sortie du conduit coudé 13c.

Un évent 20 est ménagé en partie dans la partie intermédiaire 13, à travers la partie de paroi 12 et entre cette dernière et le goulot du récipient R. Cet évent est contrôlé par le clapet de valve 16a percé en son centre et appuyant sur une saillie annulaire 13d de la partie intermédiaire 13, isolant l'orifice central du clapet de valve 16a. Celui-ci est destiné à permettre à l'air extérieur de rentrer dans le récipient R lorsque la pression à l'intérieur de celui-ci diminue suite à la sortie du liquide à travers la buse de distribution 19.

Si l'évent 20 doit assurer une fonction de barrière stérile entre l'extérieur et l'intérieur du récipient R, le canal 20 peut être remplacé par des micro-canaux en matière plastique bactéricide. On peut aussi disposer un filtre dans l'évent 20 que l'on peut incorporer en le mettant dans le moule avant l'injection des parties 11, 12, 13 de l'enceinte.

La fixation des parties de paroi 11, 12 sur la partie intermédiaire s'effectue par assemblage par enclenchement, grâce aux surfaces d'assemblage par enclenchement 11a, 12a, 13a complémentaires les unes aux autres. Un joint 17, injecté dans la même matière et en même temps que les clapets 14, 16, 16a, le joint 18 et la membrane de pompage 15 déformable de manière élastique, sert à assurer l'étanchéité entre la partie de paroi 12 et la partie intermédiaire.

Le procédé de fabrication de cette seconde forme d'exécution est le même que celui de la première forme d'exécution. Comme pour la première forme d'exécution, les trois parties 11, 12, 13 sont avantageusement reliées les une aux autres par des éléments de liaison pliables, permettant de réaliser les trois parties par une même opération de bi-injection. Par contre la présence de la buse 19 nécessite l'utilisation d'un moule à tiroir pour injecter cette buse 19.

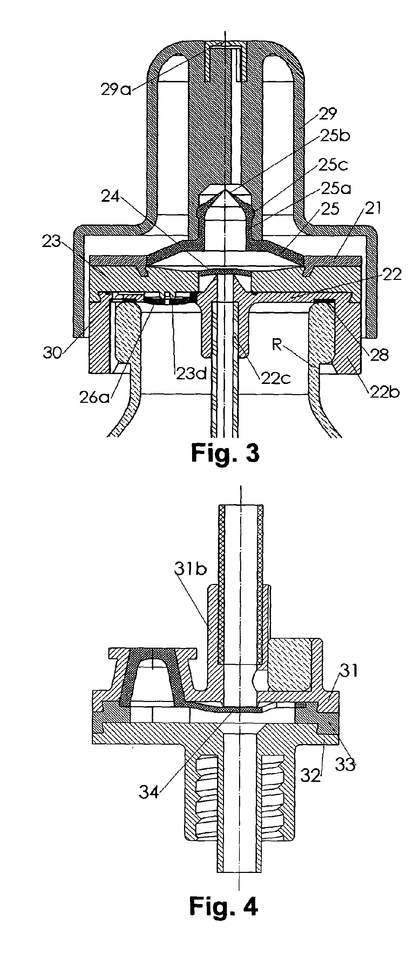

La forme d'exécution illustrée par la figure 3 ne diffère essentiellement de celle de la figure 2 que par le fait que la sortie du liquide contenu dans le récipient R se produit à travers la membrane de pompage 25, déformable de manière élastique. Cette forme d'exécution est destinée plus particulièrement à la distribution d'un produit destiné à être pulvérisé dans les narines.

A cet effet, cette membrane de pompage 25 solidaire de la partie de paroi 21 de l'enceinte comporte une partie centrale tubulaire 25a suivie d'un clapet anti-retour type «Duck bill» 25b munie d'un orifice sommital. Entre la partie tubulaire 25a et la partie conique 25b, un renflement annulaire 25c sert à la fixation d'un embout de distribution nasal 29 muni d'une buse de pulvérisation 29a. La partie conique 25b est tournée vers la buse de pulvérisation 29a, de manière que lorsque la membrane 25 est poussée vers le récipient R, une surpression est créée dans le volume de pompage de la pompe, et le liquide précédemment aspiré dans ce volume de pompage est expulsé à travers l'orifice de l'extrémité de la partie conique 25b. En relâchant la pression sur l'embout nasal 29, la membrane 25 revient dans sa position initiale et aspire du liquide du récipient R en soulevant le clapet 24.

La seconde partie de paroi 22 de l'enceinte de pompage ne comporte qu'un clapet de valve 26a percé en son centre et appuyant contre une saillie annulaire 23d de la partie intermédiaire 23 de l'enceinte de pompage. Ce clapet 26a sert à contrôler l'évent 30 ménagé de manière semblable à la forme d'exécution de la figure 2 pour permettre l'admission contrôlée d'air atmosphérique dans le récipient R pour remplacer le volume de liquide sortant par la buse 29a.

Comme dans la forme d'exécution précédente, la seconde partie de paroi 22 comporte une partie tubulaire 22b de fixation au goulot du récipient R, un conduit central d'admission 22c et un joint d'étanchéité 28 avec le goulot du récipient R.

Par contre, la partie intermédiaire 23 de cette forme d'exécution ne comporte qu'un clapet de valve 24 surmoulé en un élastomère thermoplastique ou en un élastomère silicone.

Quant à l'embout nasal 29, il s'agit évidemment d'une pièce rapportée, moulée séparément des parties 21-23 de l'enceinte de pompage.

La forme d'exécution illustrée par la figure 4 se rapporte à une enceinte multifonctions comportant un raccord Luer pour une perfusion s'écoulant pas gravité, une valve anti-retour et un accès sans aiguille. Pour empêcher l'entrée d'air une fois que le réservoir de liquide de perfusion est épuisé, une soupape de sécurité 34 est disposée dans l'enceinte étanche formée en trois parties 31-33 et est appliquée contre l'extrémité aval du conduit d'entrée 31a avec une certaine force, choisie inférieure à la force exercée par la colonne de liquide sur le clapet de la soupape 34. De ce fait, dès que la hauteur de la colonne de liquide dans le conduit d'entrée 31a descend au-dessous d'une certaine valeur, le clapet de la valve de sécurité 34 est appliqué contre l'extrémité aval du conduit 31a et empêche l'envoi d'air vers le patient.

Comme dans les formes d'exécution précédentes, les trois parties 31-34 sont injectées par bi-injection successivement d'un polypropylène ou d'un copolymère styrène-butadiène (SBC), suivie de l'injection d'un élastomère thermoplastique ou d'un élastomère silicone. Avantageusement, les trois parties sont injectées lors de la même opération d'injection, en enlevant la partie supérieure du moule, celle formant la partie de paroi 31 et en lui substituant une autre partie supérieure de moule pour former le clapet de valve de sécurité 34.

La forme d'exécution illustrée par la figure 5 se rapporte à un élément de filtrage destiné à être placé dans une ligne de transport de liquide pour le domaine médical, par exemple dans une ligne de perfusion. Ce filtre joue le rôle de barrière stérile entre un site amont, non stérile et un site aval stérile. Il peut s'agir d'un filtre pour mesurer la pression en aphérèse ou en dialyse ou encore d'un filtre pour assurer la stérilité d'un anticoagulant.

Le dispositif de filtrage illustré par la figure 5 comporte les trois parties 41, 42, 43, comme dans les formes d'exécutions précédentes, des joints d'étanchéité 47 étant surmoulés sur la partie intermédiaire 43, laquelle est elle-même surmoulée sur un filtre 49, ce qui évite les opérations d'assemblage. Avantageusement, comme précédemment, les trois parties 41, 42, 43 reliées par des éléments de liaison pliables, comme dans l'exemple illustré par la figure 6, sont injectées simultanément dans le même moule puis repliées en Z pour être assemblées par enclenchement de leurs surfaces d'assemblage complémentaires 41a, 42a, 43a.

Ce procédé de fabrication permet d'éviter l'opération de montage du filtre dans le boîtier, diminuant le temps du cycle de fabrication. Ce procédé réduit donc substantiellement le coût de fabrication d'un tel dispositif.

## Revendications

1. Enceinte étanche dont la paroi comporte deux parties (1, 2; 11, 12), qui présentent des surfaces d'assemblage respectives (1a, 2a) et une partie intermédiaire (3, 13) présentant des surfaces d'assemblage (3a) complémentaires de celles des deux parties de paroi (1, 2; 11, 12) pour réunir de manière étanche les surfaces d'assemblage complémentaires les unes aux autres, les trois parties étant en polymère injecté, **caractérisée en ce que** les trois parties (1, 2, 3; 11, 12, 13) sont en un polymère rigide, l'une d'entre elles au moins présentant au moins une surface de liaison permanente avec au moins un élément (4-7; 14-17) qui est en un élastomère thermoplastique ou en un élastomère silicone.

2. Enceinte selon la revendication 1, comportant une ouverture d'admission (1b) et une ouverture de sortie (2c) une desdites parties (1-3) comportant au moins un élément (4; 14; 24; 34) pour interrompre temporairement ou non la communication entre les deux ouvertures à travers l'enceinte.

3. Enceinte selon la revendication 2, dans laquelle une des deux parties de la paroi de l'enceinte comporte l'ouverture d'admission et des moyens de fixation à un récipient et l'autre partie de cette paroi comporte un élément mobile d'entraînement de fluide, la partie intermédiaire comporte une buse de pulvérisation, un conduit entre cette buse et le volume de l'enceinte adjacent audit élément mobile d'entraînement de fluide, un élément de contrôle de l'écoulement de fluide entre ledit volume adjacent à l'élément mobile d'entraînement de fluide et la buse et un élément de contrôle de l'écoulement de fluide entre l'amont de l'ouverture d'admission et le volume adjacent à l'élément mobile d'entraînement de fluide.

4. Enceinte étanche selon la revendication 1, comportant une ouverture d'admission (41b) et une ouverture de sortie (42c), dans laquelle la partie intermédiaire (43) est venue de moulage avec un filtre (49)

5. Enceinte selon l'une des revendications précédentes, dans laquelle des éléments de liaison pliables (8) relient les différentes parties de l'enceinte les unes aux autres, de manière à permettre de réunir les surfaces d'assemblage par rabattement des parties de l'enceinte les unes sur les autres.

6. Enceinte selon l'une des revendications 2 et 4, dans laquelle une des deux parties de la paroi de l'enceinte comporte l'ouverture d'admission et des moyens de fixation à un récipient et l'autre partie de cette paroi comporte un élément déformable de manière élastique pour l'entraînement de fluide et qui comporte une partie centrale munie de moyens de fixation d'un embout de pulvérisation nasal, cette partie centrale présentant une partie conique muni d'un orifice de sortie sommital et dont la conicité est dirigée vers l'extérieur de l'enceinte.

7. Enceinte selon l'une des revendications précédentes, dans laquelle les surfaces d'assemblage respectives des deux parties de paroi et les surfaces d'assemblage complémentaires de la partie intermédiaire, sont des surfaces d'assemblage par enclenchement.

8. Procédé de fabrication de l'enceinte étanche selon la revendication 1, selon lequel la paroi en polymère rigide et ledit élément en élastomère thermoplastique ou en élastomère silicone sont injectés successivement selon la technique de bi-injection.

9. Procédé selon la revendication 8, selon lequel on place un élément (49) à l'intérieur du moule et on surmoule le polymère rigide sur cet élément placé à l'intérieur du moule, de manière à l'intégrer à la paroi de l'enceinte.

10. Procédé selon l'une des revendications 8 et 9 selon lequel on moule les parois de l'enceinte en trois parties (1, 2, 3) reliées entre elles et on plie ensuite ces trois parties en Z pour les assembler.
